# EUROPEAN PATENT APPLICATION

(11) **EP 4 711 474 A1**
(43) Date of publication of application: **18.03.2026**
(21) Application number: 24926640.4
(22) Date of filing: 01.08.2024
(51) Int. Cl.: C12Q 1/6888, C12Q 1/6851, C12N 15/12, C12N 15/11

(54) **DNA FRAGMENTS, PRIMER SET AND KIT FOR GENETIC SEX IDENTIFICATION OF ACIPENSER SINENSIS, AND USES**

(30) Priority: 29.02.2024 CN 202410231404
(71) Applicant: Chinese Sturgeon Research Institute of CTG, Yichang, Hubei 443100 (CN)
(72) Inventor: LI, Sha, Yichang, Hubei 443100 (CN); HU, Yacheng, Yichang, Hubei 443100 (CN); JIANG, Wei, Yichang, Hubei 443100 (CN); SU, Wei, Yichang, Hubei 443100 (CN); LIU, Xueqing, Yichang, Hubei 443100 (CN); DU, Hejun, Yichang, Hubei 443100 (CN); WANG, Dianchang, Yichang, Hubei 443100 (CN)
(74) Representative: Groth & Co. KB
(86) International application number: PCT/CN2024/109249
(87) International publication number: WO 2025/179772

(57) **Abstract**

Provided are DNA fragments, a primer set and a kit for genetic sex identification of *Acipenser sinensis,* and uses. In a first aspect, provided is a use of a DNA fragment common to both male and female *Acipenser sinensis* and a female *Acipenser sinensis-specific* DNA fragment in genetic sex identification of *Acipenser sinensis,* wherein the nucleotide sequence of the DNA fragment common to both male and female *Acipenser sinensis* is as shown in SEQ ID NO: 1, and the nucleotide sequence of the female *Acipenser sinensis-specific* DNA fragment is as shown in SEQ ID NO:2. The two DNA fragments provided are used as target amplification fragments, and genetic sex identification is carried out on *Acipenser sinensis* by means of environmental DNA samples, thereby realizing non-contact genetic sex identification and achieving good stability and accuracy.

## Description

### CROSS REFERENCE TO RELATED APPLICATIONS

The present application claims the benefit of priority to the Chinese Patent Application No. 202410231404.5 filed with China National Intellectual Property Administration on February 29, 2024 and entitled "DNA Fragments, Primer Set and Kit for Genetic Sex Identification of *Acipenser Sinensis,* and Uses", which is incorporated herein by reference in its entirety.

### TECHNICAL FIELD

The present disclosure relates to the technical field of genetic sex identification of *Acipenser sinensis,* and in particular to DNA fragments, a primer set and a kit for genetic sex identification of *Acipenser sinensis,* and uses.

### BACKGROUND

*Acipenser sinensis* (known as Chinese sturgeon) is a typical anadromous fish species. During its breeding season from September to November each year, mature individuals migrate upstream along the Yangtze River to spawn in the upper reaches. The hatchlings migrate downstream to the estuary of the Yangtze River, where they undergo feeding and growth before entering the ocean. Upon reaching sexual maturity, they return to the Yangtze River to reproduce. Due to the construction of hydroelectric projects and other human activities, the population of *Acipenser sinensis* has declined sharply. In order to preserve the fish species, many Chinese research institutions have implemented various conservation measures for *Acipenser sinensis,* including ex-situ conservation and artificial propagation and release programs.

However, there are no significant external morphological differences between male and female *Acipenser sinensis.* They lack nuptial coloration during the breeding season, and both sexes lack external genitalia or secondary sexual characteristics such as nuptial tubercles, making sex identification extremely difficult.

Previously, several sex identification methods, including B-scan ultrasonography, endoscopy, and molecular identification, have been developed. However, these previous sex identification methods either require the fish to reach a relatively old age or may cause harm to the fish. For example, the B-scan ultrasonography method requires individuals of *Acipenser sinensis* older than five years; and the endoscopy requires an abdominal incision and achieves high accuracy only in fish older than five years. Although molecular methods can determine sex at any developmental stage, they require the collection of tissue samples.

Organisms release their own DNA into the surrounding environment through various routes, such as skin, mucus, urine, feces, blood, sperms and eggs, and decaying carcasses. This DNA is referred to as environmental DNA. Unlike DNA extracted directly from biological tissues, environmental DNA exists in different ways and is prone to degradation to varying degrees. Therefore, a target sequence should not be too long. However, existing target sequences mostly used for species identification, and there have been no reports to date on genetic sex identification *of Acipenser sinensis* using environmental DNA samples.

### SUMMARY

The present disclosure provides DNA fragments, a primer set and a kit for genetic sex identification of *Acipenser sinensis,* and uses.

In a first aspect, the present disclosure provides use of a DNA fragment common to both male and female *Acipenser sinensis* and a female *Acipenser sinensis-specific* DNA fragment in genetic sex identification of *Acipenser sinensis,* wherein a nucleotide sequence of the DNA fragment common to both male and female *Acipenser sinensis* is as shown in SEQ ID NO: 1, and a nucleotide sequence of the female *Acipenser sinensis-specific* DNA fragment is as shown in SEQ ID NO:2.
Based on the genomic information and differential loci between male and female individuals of *Acipenser sinensis,* the present disclosure provides a DNA fragment common to both male and female *Acipenser sinensis* (a nucleotide sequence of the same is as shown in SEQ ID NO:1), and the DNA fragment can be stably and efficiently amplified in individuals of both sexes and at all developmental stages of *Acipenser sinensis,* and may serve as a target sequence for amplification to identify whether an environmental DNA sample contains DNA of *Acipenser sinensis.* The present disclosure further provides a female *Acipenser sinensis-specific* DNA fragment (a nucleotide sequence of the same is as shown in SEQ ID NO:2), and the fragment is specific to female *Acipenser sinensis* and absent in male *Acipenser sinensis,* and may serve as a target amplification sequence to identify the genetic sex *of Acipenser sinensis,* thereby improving detection accuracy.

In a second aspect, the present disclosure provides, based on the DNA fragment common to both male and female *Acipenser sinensis* and the female *Acipenser sinensis-specific* DNA fragment provided in the first aspect, a primer set for amplifying a DNA fragment common to both male and female *Acipenser sinensis* and a female *Acipenser sinensis-specific* DNA fragment, which includes a first primer and a second primer for amplifying the DNA fragment common to both male and female *Acipenser sinensis,* and a third primer and a fourth primer for amplifying the female *Acipenser sinensis-specific* DNA fragment; specifically
a nucleotide sequence of the first primer is as shown in SEQ ID NO:3;
a nucleotide sequence of the second primer is as shown in SEQ ID NO:4;
a nucleotide sequence of the third primer is as shown in SEQ ID NO:5; and
a nucleotide sequence of the fourth primer is as shown in SEQ ID NO:6.

It should be noted that the first primer provided in the present disclosure is a reverse primer for amplifying the DNA fragment common to both male and female *Acipenser sinensis,* and the second primer is a forward primer for amplifying the DNA fragment common to both male and female *Acipenser sinensis;* and the third primer is a reverse primer for amplifying the female *Acipenser sinensis-specific* DNA fragment, and the fourth primer is a forward primer for amplifying the female *Acipenser sinensis-specific* DNA fragment.

In a third aspect, the present disclosure provides a kit for genetic sex identification of *Acipenser sinensis,* which includes the primer set provided in the second aspect.

The kit described above further includes a negative control.

In a fourth aspect, the present disclosure provides use of the primer set provided in the second aspect or the kit provided in the third aspect in genetic sex identification of *Acipenser sinensis.*

In a fifth aspect, the present disclosure provides a method for genetic sex identification of *Acipenser sinensis,* including the following steps:
collecting a DNA sample from the *Acipenser sinensis* to be tested;
designing a first primer and a second primer for amplifying the DNA fragment common to both male and female *Acipenser sinensis,* and a third primer and a fourth primer for amplifying the female *Acipenser sinensis-specific* DNA fragment according to the DNA fragment common to both male and female *Acipenser sinensis* and the female *Acipenser sinensis-specific* DNA fragment provided in the first aspect;
performing a first real-time quantitative polymerase chain reaction (PCR) detection on the DNA sample using the first primer and the second primer, and performing a second real-time quantitative PCR detection on the DNA sample using the third primer and the fourth primer; and
determining a genetic sex of the *Acipenser sinensis* to be tested based on results of the first and second real-time quantitative PCR detections.

In a specific embodiment, the method includes the following steps:
step 1. collecting a DNA sample from the *Acipenser sinensis* to be tested.

First, the DNA sample to be tested is collected from the *Acipenser sinensis,* and the DNA sample is an environmental DNA sample. It should be understood that when the environmental DNA sample is derived from a water sample inhabited by *Acipenser sinensis,* it is necessary to ensure that only a single individual *Acipenser sinensis* is present in the water sample. For example, the individual *Acipenser sinensis* to be tested may be temporarily kept in a separate tank. After DNA has accumulated in the water, the water sample is collected for subsequent DNA extraction. For wild *Acipenser sinensis,* body mucus or feces of the *Acipenser sinensis* may be collected as the environmental DNA samples.

Based on the collected samples to be tested, DNA extraction is then performed using conventional techniques. In a specific embodiment, taking the water sample from the habitat of the *Acipenser sinensis* as an example, the DNA extraction process includes the following steps: first, the collected water sample is filtered using a filter membrane, which may be performed using a vacuum filtration device. Then, blank edges of each filter membrane are trimmed, the filter membrane is then cut along a center line into four fan-shaped sectors of equal area, and each sector is placed into a 50 mL centrifuge tube containing 3 mL of SLX reaction solution and 500 mg of glass beads, the centrifuge tube is vortexed at a maximum speed using a vortex mixer for mixing, followed by heating and sonication in an ultrasonic device, with intermittent vortexing during the process, such that all substances on the filter membrane are eluted into the SLX reaction solution, maximizing the extraction of DNA from the filter membrane. DNA is subsequently extracted using a DNA extraction kit, and the extracted DNA is stored at -20 °C for later use.

Furthermore, during sonication, appropriate heating may be applied, with a temperature not exceeding 50 °C, to shorten the elution time of substances on the filter membrane.

Step 2. a first primer and a second primer for amplifying the DNA fragment common to both male and female *Acipenser sinensis,* and a third primer and a fourth primer for amplifying the female *Acipenser sinensis-specific* DNA fragment are designed according to the DNA fragment common to both male and female *Acipenser sinensis* and the female *Acipenser sinensis-specific* DNA fragment.

In a specific embodiment, the primers provided in the second aspect of the present disclosure may be used for subsequent amplification of DNA fragment.

Step 3. A first real-time quantitative polymerase chain reaction (PCR) detection is performed on the DNA sample using the first primer and the second primer, and a second real-time quantitative PCR detection is performed on the DNA sample using the third primer and the fourth primer; and
In the present disclosure, the sequence of the first and second real-time quantitative PCR detections is not limited, that is, the quantitative PCR detection may be performed on the DNA sample first using the first and second primers, and then the DNA sample may be subjected to the quantitative PCR detection using the third and fourth primers, or alternatively, the quantitative PCR detection may be performed on the DNA sample first using the third and fourth primers, and then the DNA sample may be subjected to the quantitative PCR detection using the first and second primers.

The real-time quantitative PCR detection may be performed using conventional techniques. The present disclosure mainly focuses on the interpretation of detection results: when Ct values of both the first and second real-time quantitative PCR detection results are less than or equal to 36, it is determined that the genetic sex of the *Acipenser sinensis* is female; when a Ct value of the second real-time quantitative PCR detection result is greater than 36 or shows "undetermined", and a Ct value of the first real-time quantitative PCR detection result is less than or equal to 36, it is determined that the genetic sex of the *Acipenser sinensis* is male; and when Ct values of both the first and second real-time quantitative PCR detection results are greater than 36 or show "undetermined", it is determined that the DNA sample does not contain DNA from the *Acipenser sinensis.*

It should be understood that, when the DNA samples are subjected to the real-time quantitative PCR detection, the real-time quantitative PCR detection is also performed on a negative control using the same method. The negative control may be sterile water.

In summary, the DNA fragment common to both male and female *Acipenser sinensis* and the female *Acipenser sinensis-specific* DNA fragment in genetic sex identification of *Acipenser sinensis* common to both male and female *Acipenser sinensis* provided in the present disclosure can be used for genetic sex identification of *Acipenser sinensis.* This method does not cause any harm to *Acipenser sinensis* and effectively avoids impact on breeding and aquaculture during sex identification. Moreover, the relatively short target sequences help prevent the detection failure due to environmental DNA degradation, enabling rapid and accurate identification of genetic sex in individual *Acipenser sinensis* of different ages. This method is applicable to *Acipenser sinensis* throughout their entire life cycle.

### BRIEF DESCRIPTION OF THE DRAWINGS

FIG. 1a illustrates real-time PCR amplification plots obtained from real-time quantitative PCR detection of female *Acipenser sinensis* samples using a primer pair Co-Nuc-FM common to both male and female *Acipenser sinensis* according to Example 1 of the present disclosure.
FIG. 1b illustrates melt curves obtained from real-time quantitative PCR of female *Acipenser sinensis* samples using a primer pair Co-Nuc-FM common to both male and female *Acipenser sinensis* according to Example 1 of the present disclosure.
FIG. 2a illustrates real-time PCR amplification plots obtained from real-time quantitative PCR of male *Acipenser sinensis* samples using a primer pair Co-Nuc-FM common to both male and female *Acipenser sinensis* according to Example 1 of the present disclosure.
FIG. 2b illustrates melt curves obtained from real-time quantitative PCR of male *Acipenser sinensis* samples using a primer pair Co-Nuc-FM common to both male and female *Acipenser sinensis* according to Example 1 of the present disclosure.
FIG. 3a illustrates real-time PCR amplification plots obtained from real-time quantitative PCR of female *Acipenser sinensis* samples using a female-specific primer pair Spec-Nuc-F according to Example 1 of the present disclosure.
FIG. 3b illustrates melt curves obtained from real-time quantitative PCR of female *Acipenser sinensis* samples using a female-specific primer pair Spec-Nuc-F according to Example 1 of the present disclosure.
FIG. 4a illustrates real-time PCR amplification plots obtained from real-time quantitative PCR of male *Acipenser sinensis* samples using a female-specific primer pair Spec-Nuc-F according to Example 1 of the present disclosure.
FIG. 4b illustrates melt curves obtained from real-time quantitative PCR of male *Acipenser sinensis* samples using a female-specific primer pair Spec-Nuc-F according to Example 1 of the present disclosure.
FIG. 5a illustrates real-time PCR amplification plots obtained from real-time quantitative PCR detection of a female *Acipenser sinensis* sample using a primer pair Co-Nuc-FM common to both male and female *Acipenser sinensis* according to Example 2 of the present disclosure.
FIG. 5b illustrates melt curves obtained from real-time quantitative PCR of a female *Acipenser sinensis* sample using a primer pair Co-Nuc-FM common to both male and female *Acipenser sinensis* according to Example 2 of the present disclosure.
FIG. 6a illustrates real-time PCR amplification plots obtained from real-time quantitative PCR of a male *Acipenser sinensis* sample using a primer pair Co-Nuc-FM common to both male and female *Acipenser sinensis* according to Example 2 of the present disclosure.
FIG. 6b illustrates melt curves obtained from real-time quantitative PCR of a male *Acipenser sinensis* sample using a primer pair Co-Nuc-FM common to both male and female *Acipenser sinensis* according to Example 2 of the present disclosure.
FIG. 7a illustrates real-time PCR amplification plots obtained from real-time quantitative PCR detection of a negative control sample using a primer pair Co-Nuc-FM common to both male and female *Acipenser sinensis* according to Example 2 of the present disclosure.
FIG. 7b illustrates melt curves obtained from real-time quantitative PCR of a negative control sample using a primer pair Co-Nuc-FM common to both male and female *Acipenser sinensis* according to Example 2 of the present disclosure.
FIG. 8a illustrates real-time PCR amplification plots obtained from real-time quantitative PCR of a female *Acipenser sinensis* sample using a female-specific primer pair Spec-Nuc-F according to Example 2 of the present disclosure.
FIG. 8b illustrates melt curves obtained from real-time quantitative PCR of a female *Acipenser sinensis* sample using a female-specific primer pair Spec-Nuc-F according to Example 2 of the present disclosure.
FIG. 9a illustrates real-time PCR amplification plots obtained from real-time quantitative PCR of a male *Acipenser sinensis* sample using a female-specific primer pair Spec-Nuc-F according to Example 2 of the present disclosure.
FIG. 9b illustrates melt curves obtained from real-time quantitative PCR of a male *Acipenser sinensis* sample using a female-specific primer pair Spec-Nuc-F according to Example 2 of the present disclosure.
FIG. 10a illustrates real-time PCR amplification plots obtained from real-time quantitative PCR detection of a negative control sample using a female-specific primer pair Spec-Nuc-F according to Example 2 of the present disclosure.
FIG. 10b illustrates melt curves obtained from real-time quantitative PCR of a negative control sample using a female-specific primer pair Spec-Nuc-F according to Example 2 of the present disclosure.
FIG. 11a illustrates real-time PCR amplification plots obtained from real-time quantitative PCR detection of a two-fold diluted water sample using a female-specific primer pair Spec-Nuc-F according to Example 3 of the present disclosure.
FIG. 11b illustrates melt curves obtained from real-time quantitative PCR detection of a two-fold diluted water sample using a female-specific primer pair Spec-Nuc-F according to Example 3 of the present disclosure.
FIG. 12 is a diagram illustrating sensitivity detection of a female-specific primer pair Spec-Nuc-F according to Example 3 of the present disclosure.

### DETAILED DESCRIPTIONS OF THE EMBODIMENTS

In order to make the objectives, technical solutions and advantages of the embodiments of the present disclosure clearer, the technical solutions of the embodiments of the present disclosure will be described clearly and completely with reference to the accompanying drawings of the embodiments of the present disclosure. Apparently, the described embodiments are only some, but not all, embodiments of the present disclosure. On the basis of the embodiments of the present disclosure, all other embodiments obtained by those of ordinary skill in the art without inventive efforts shall fall within the scope of protection of the present disclosure.

### Example 1 Verification of Genetic Sex Specificity of Female-Specific Primer Pairs

Step 1. Three male and three female *Acipenser sinensis* of known genetic sex were selected, respectively, and fin clips were collected for DNA extraction.

Step 2. Quantitative polymerase chain reaction (PCR) detection was performed on the female *Acipenser sinensis* and the male *Acipenser sinensis* using a primer pair Co-Nuc-FM for amplifying the DNA fragment common to both male and female *Acipenser sinensis* and a primer pair Spec-Nuc-F for amplifying the female *Acipenser sinensis-specific* DNA fragment, respectively. An amplification system had a total volume of 20 µL, which consisted of 10 µL of 2× Q3 SYBR qPCR Master Mix (Universal), 0.4 µL of each forward and reverse primer, 1 µL of DNA template, and 8.2 µL of ddH₂O. Reaction conditions were as follows: initial denaturation at 95 °C for 30 s; denaturation at 95 °C for 10 s; annealing at 56 °C for 30 s; extension at 72 °C for 30 s; 40 cycles in total.

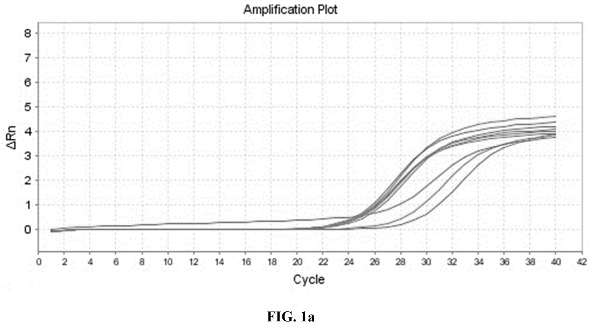

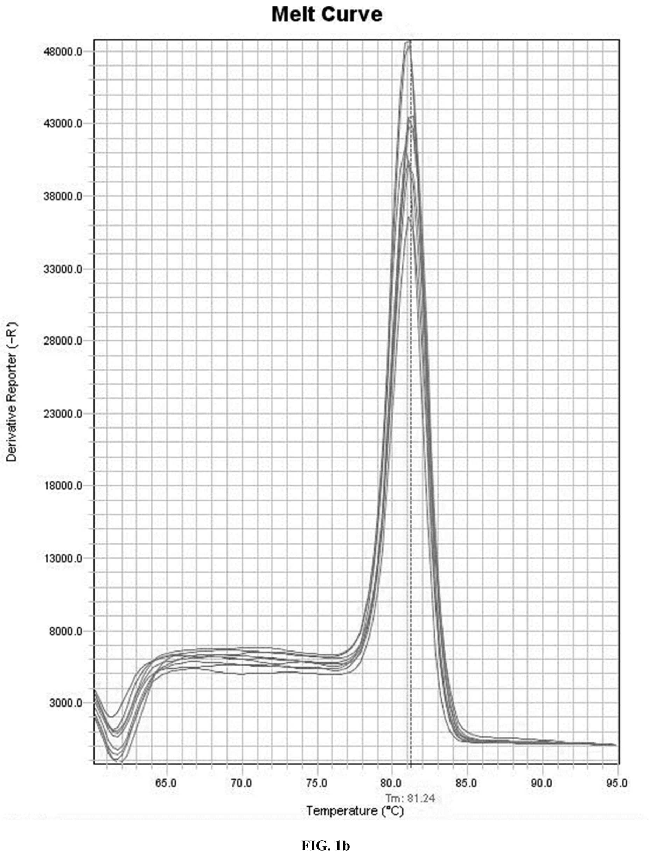

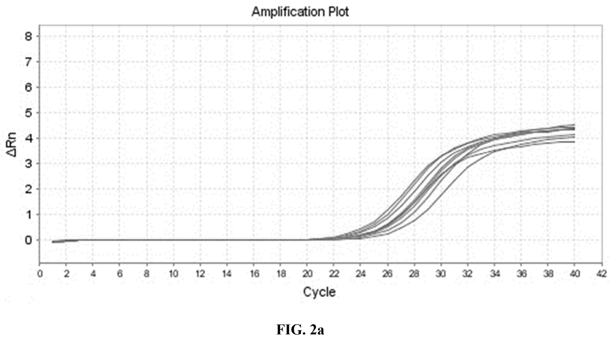

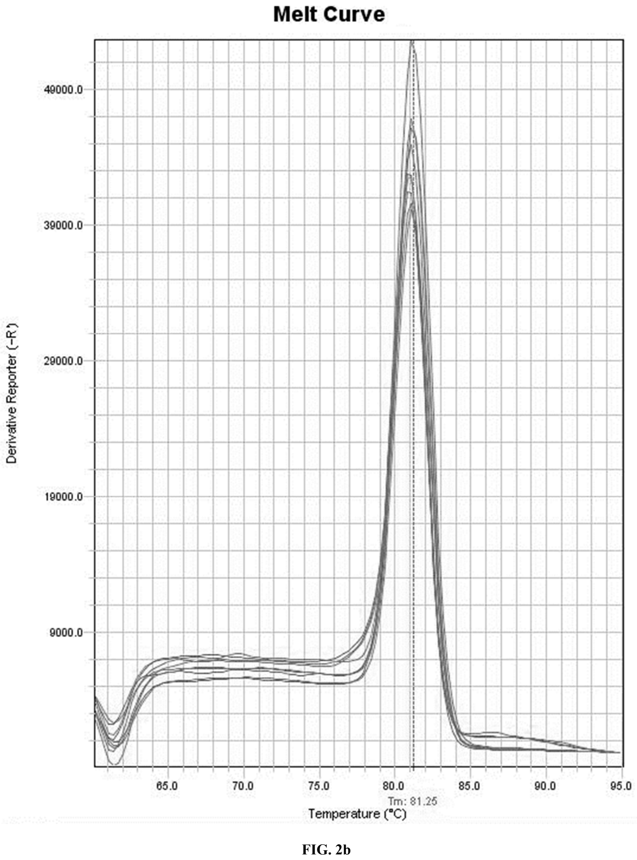

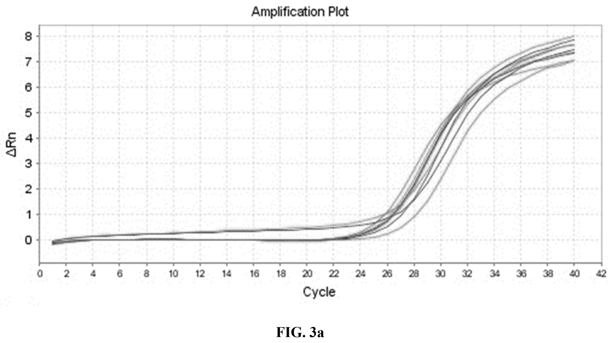

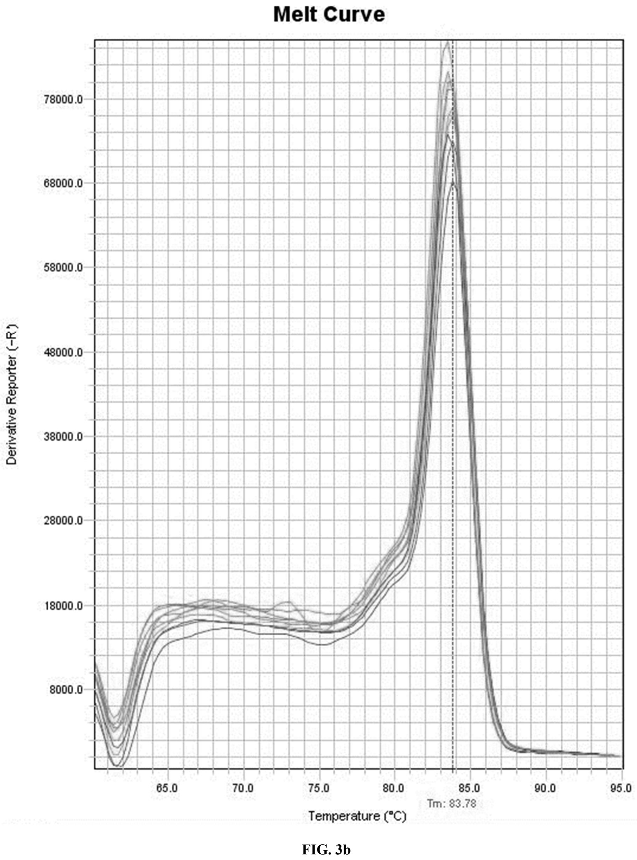

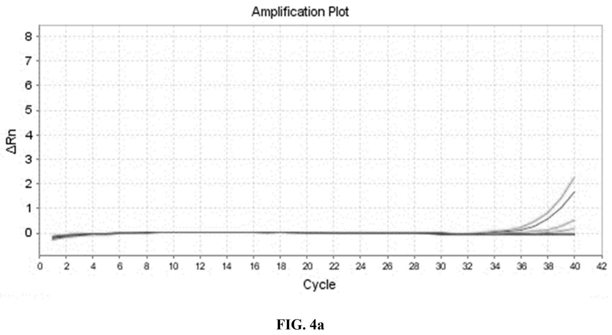

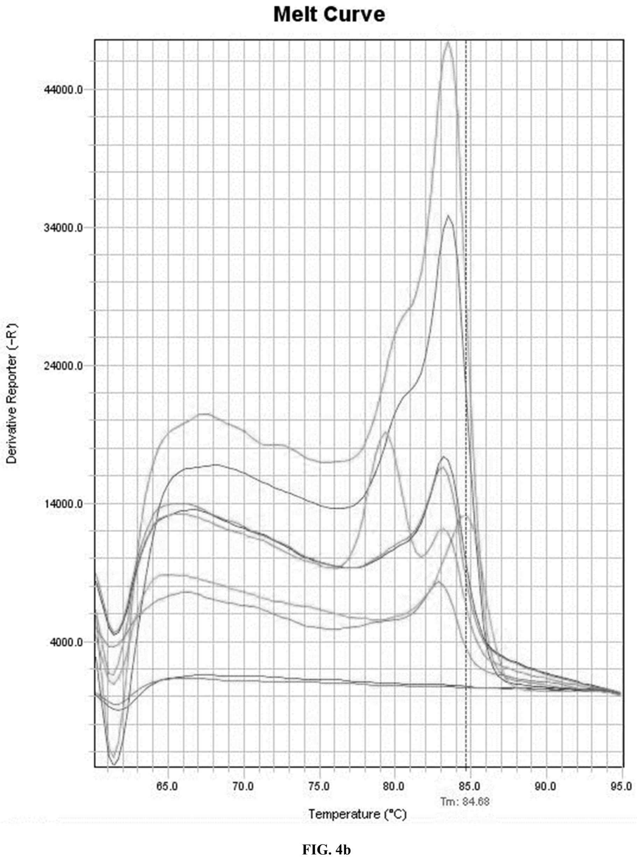

PCR amplification results are shown in FIGS. 1a-4b. For the primer pair Co-Nuc-FM for amplifying the DNA fragment common to both male and female *Acipenser sinensis,* Ct values in both female and male *Acipenser sinensis* samples were less than or equal to 36, indicating positive amplification. For the primer pair for amplifying the female *Acipenser sinensis-specific* DNA fragment, Ct values in the female *Acipenser sinensis* samples were less than or equal to 36, indicating positive amplification, while Ct values in the male *Acipenser sinensis* samples were greater than 36 or undetermined, indicating negative amplification. The results confirmed that the primer pair common to both male and female *Acipenser sinensis* showed good amplification efficiency on both female and male *Acipenser sinensis,* while the female-specific primer pair achieves positive amplification only in female *Acipenser sinensis,* demonstrating good genetic sex specificity.

### Example 2: Use of Two Primer Pairs in Individual Acipenser sinensis Water Samples

Step 1. One female and one male *Acipenser sinensis,* each approximately one year old, were selected from an *Acipenser sinensis* aquaculture pond and temporarily placed in separate aquaria. After 12 h, 2 L of water sample was collected from each aquarium. The water samples were subjected to vacuum filtration through a 0.22 µm filter membrane, and one filter membrane was used for filtering one liter of water. The filter membranes after filtering were placed into centrifuge tubes and stored at -20 °C for later use. Throughout the operation, gloves were worn, and filter membranes were transferred using forceps. Prior to filtering different samples, the filter was rinsed with sterile water to prevent cross-contamination. Sterile water was used as a negative control.

Step 2. Blank edges of each filter membrane were trimmed, the filter membrane was then cut along a center line into four fan-shaped sectors of equal area, and each sector was placed into a 50 mL centrifuge tube containing 3 mL of SLX reaction solution and 500 mg of glass beads, the centrifuge tube was vortexed at a maximum speed using a vortex mixer for mixing, followed by heating and sonication in an ultrasonic device, with intermittent vortexing during the process. DNA was then extracted from the filter membranes using the E.Z.N.A.^{®} Water DNA Kit (Omega, USA). The extracted DNA was stored at -20 °C for later use.

Step 3. Quantitative PCR detection was performed on the aforementioned water samples and negative sample using a primer pair Co-Nuc-FM for amplifying the DNA fragment common to both male and female *Acipenser sinensis.* An amplification system had a total volume of 20 µL, which consisted of 10 µL of 2× Q3 SYBR qPCR Master Mix (Universal), 0.4 µL of each forward and reverse primer, 1 µL of DNA template, and 8.2 µL of ddH₂O. Reaction conditions were as follows: initial denaturation at 95 °C for 30 s; denaturation at 95 °C for 10 s; annealing at 56 °C for 30 s; extension at 72 °C for 30 s; 40 cycles in total.

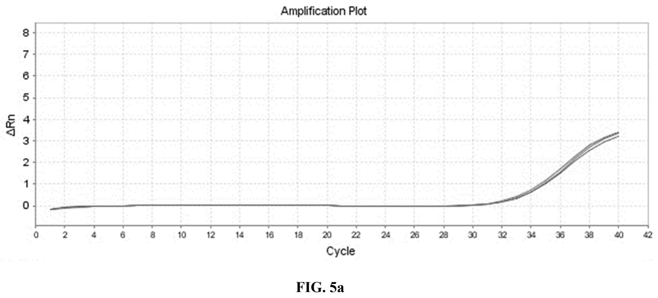

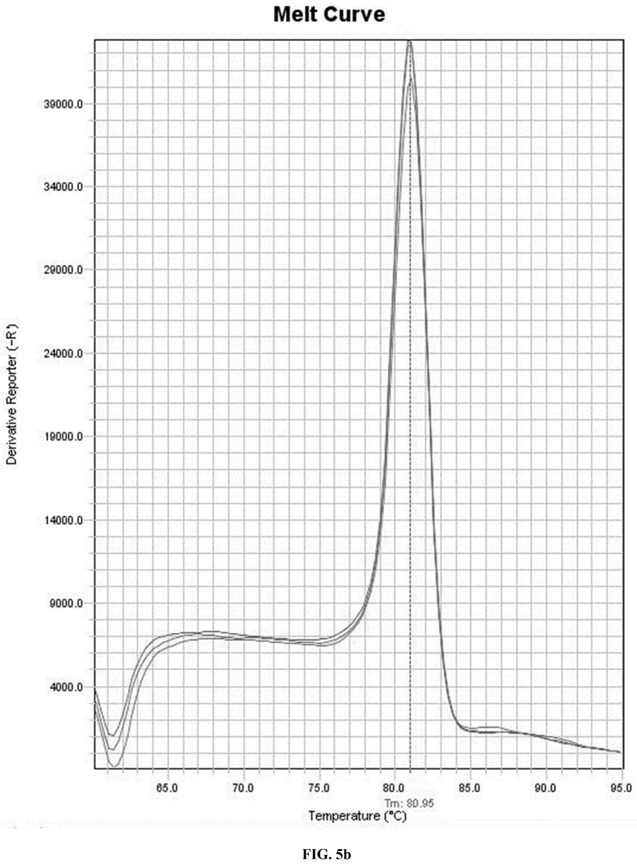

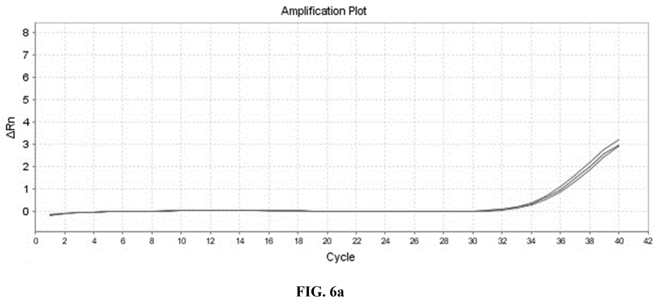

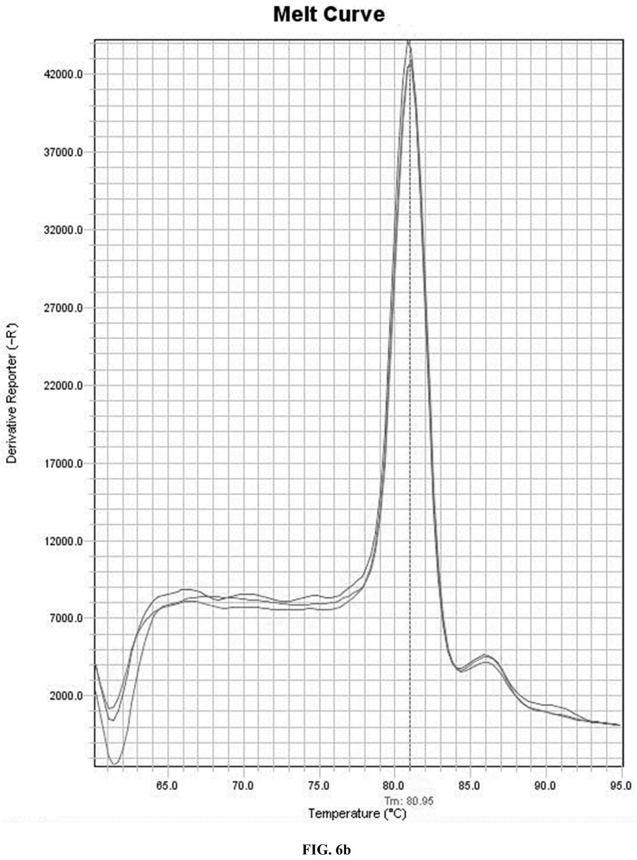

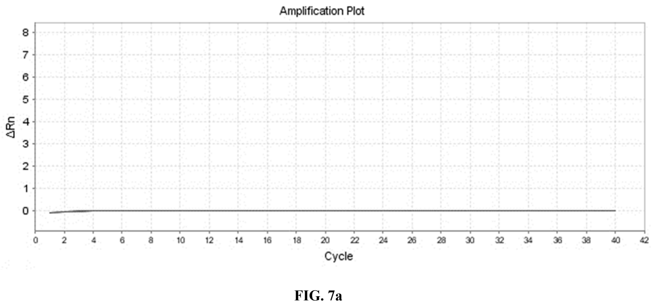

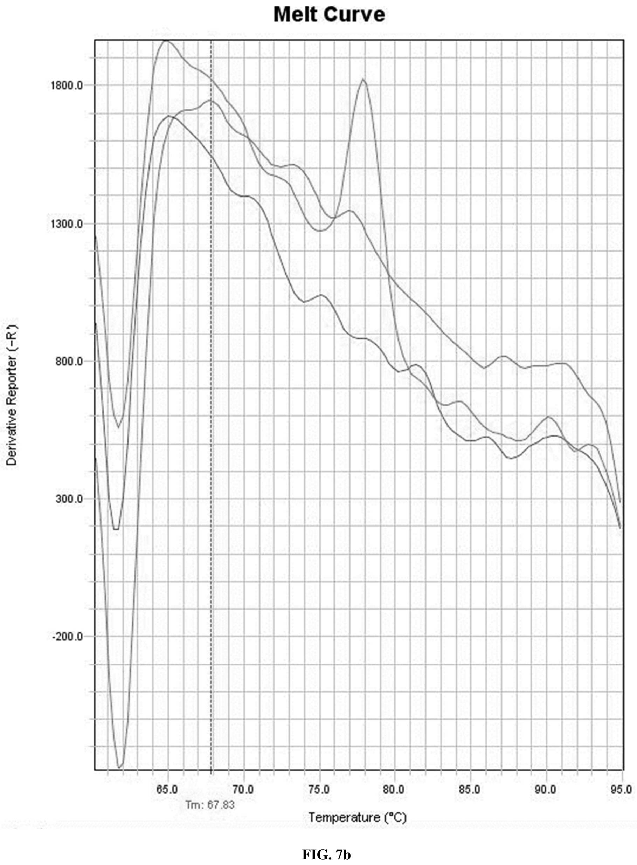

The amplification results are shown in FIGS. 5a-7b. Ct values of the primer pair Co-Nuc-FM common to both male and female *Acipenser sinensis* in the two water samples were both less than or equal to 36, indicating positive amplification and confirming the presence of *Acipenser sinensis* in the sampled water. Ct values of the negative control were greater than 36 or undetermined, indicating negative amplification.

Step 4. Quantitative PCR detection was performed on the aforementioned water samples and negative sample using a primer pair Spec-Nuc-F for amplifying the female *Acipenser sinensis-specific* DNA fragment, and the amplification system and conditions were the same as those in the Step 3.

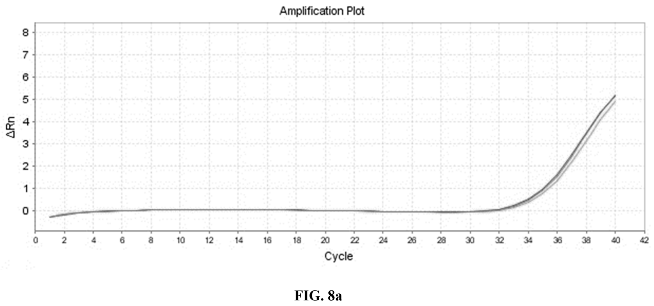

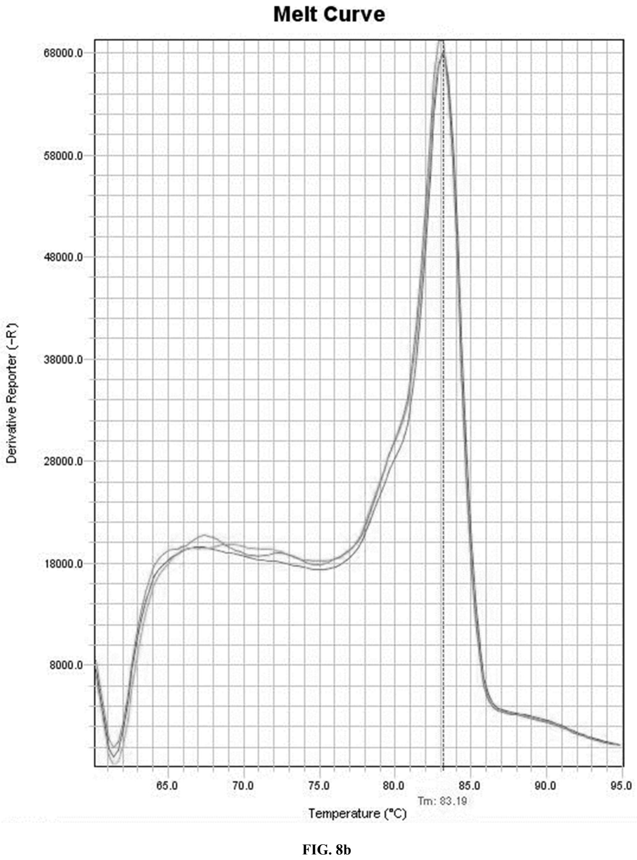

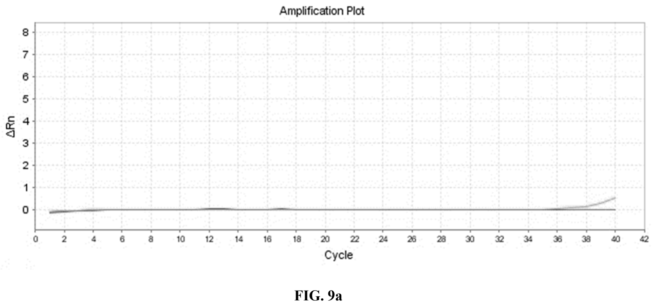

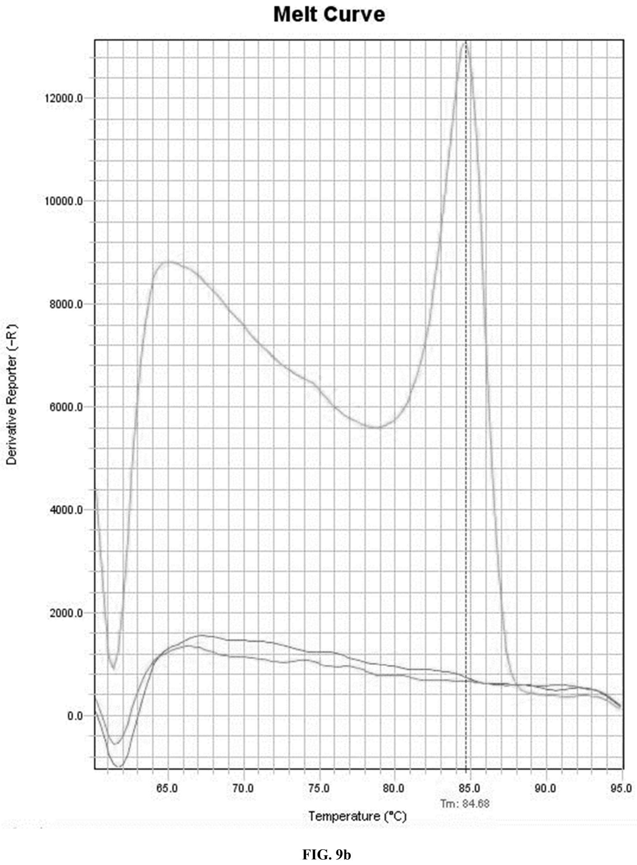

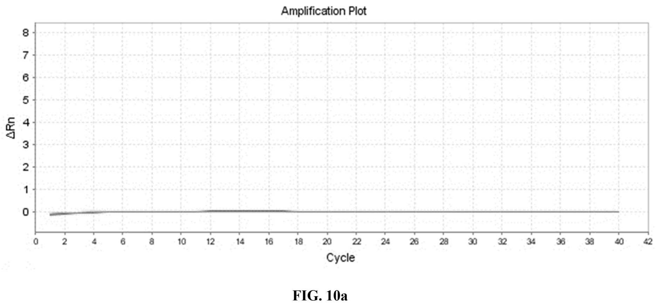

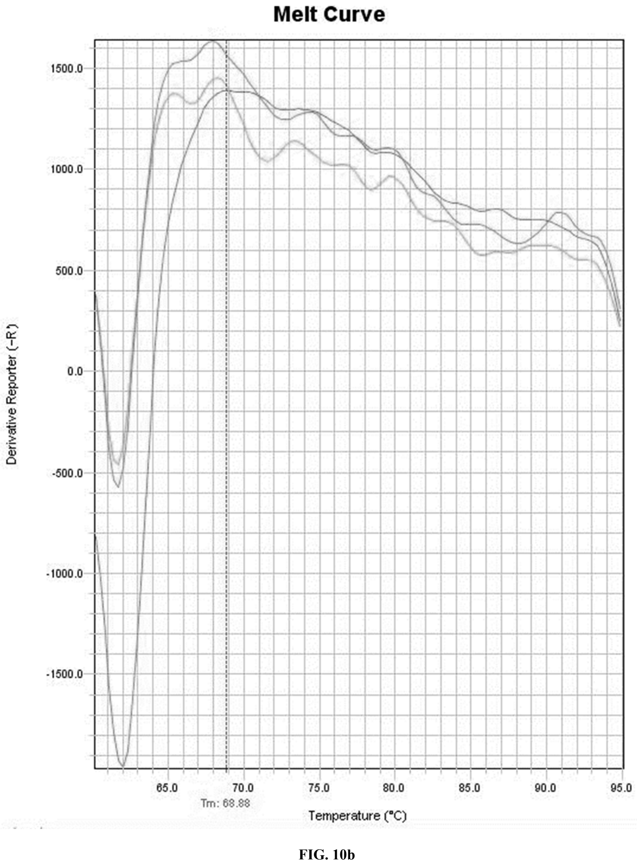

The amplification results are shown in FIGS. 8a-10b. Ct values of the female-specific primer pair Spec-Nuc-F in the female *Acipenser sinensis* water sample were both less than or equal to 36, indicating positive amplification and confirming the genetic sex of the *Acipenser sinensis* as female; Ct values in the male *Acipenser sinensis* water sample were greater than 36 or undetermined, indicating negative amplification and confirming the genetic sex of the *Acipenser sinensis* as male; and the genetic sex identification results using the two primer pairs were consistent with the actual genetic sex of the *Acipenser sinensis,* demonstrating that these two primer pairs can successfully identify the genetic sex of individual *Acipenser sinensis* through water samples.

### Example 3 Sensitivity Detection of Primer Pairs

As demonstrated in the two examples above, both primer pairs can realize amplification reaction stably in positive samples. This example focuses on evaluating the sensitivity of the female-specific primer pair.

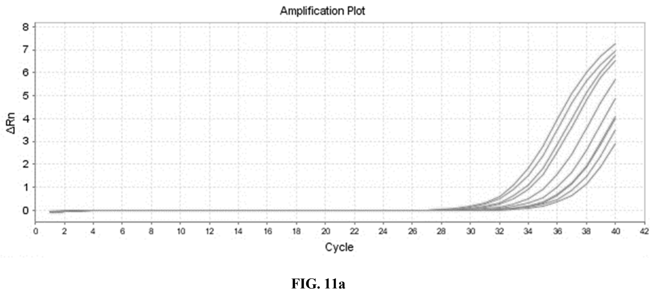

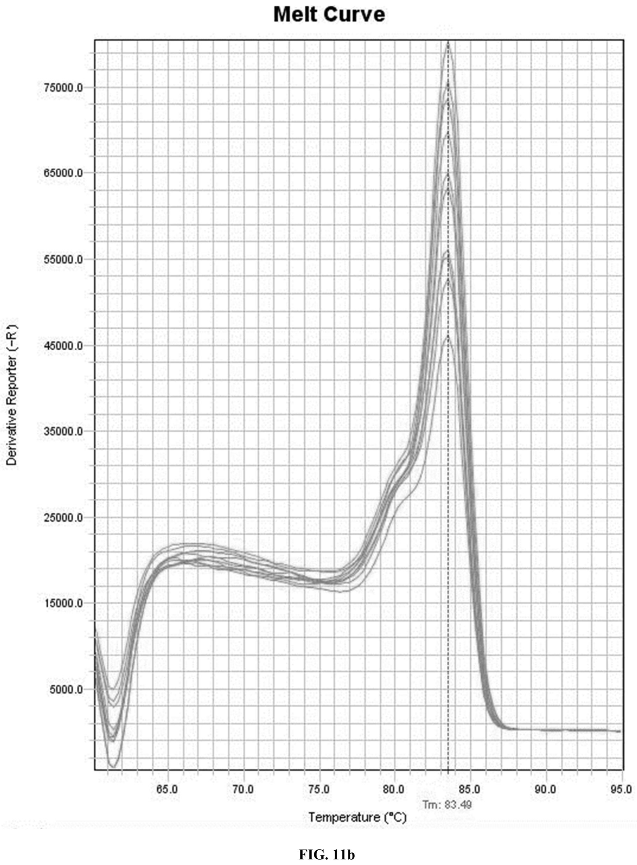

DNA extracted from a water sample collected from one aquaculture pond was subjected to a two-fold serial dilution. A first dilution was 1/2 of an initial concentration, and a second dilution was 1/4 of the initial concentration, and so on, generating four diluted solutions. PCR amplification was performed using the reaction system and conditions same as those in Example 2. The amplification results are shown in FIGS. 11a-11b.

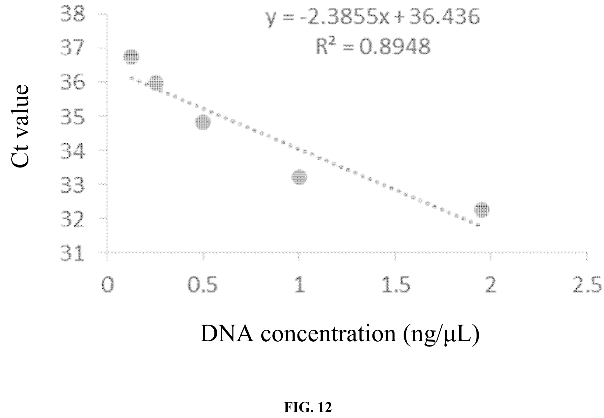

As shown in FIGS. 11a-11b, the initial DNA concentration of the water sample was 2.1 ng/µL. At an eight-fold dilution, a Ct value approached 36, and at a 16-fold dilution, a Ct value was less than or equal to 36. Based on the initial DNA concentration, the DNA concentrations corresponding to different dilution factors were calculated using two-fold decrements. A linear relationship between the Ct value and the DNA concentration was then plotted. As shown in FIG. 12, a minimum detectable effective DNA concentration of the female-specific primer pair was approximately 0.25 ng/µL.

In summary, the DNA fragment common to both male and female *Acipenser sinensis* and the female *Acipenser sinensis-specific* DNA fragment provided in the present disclosure can be used for genetic sex identification of *Acipenser sinensis.* This method does not cause any harm to *Acipenser sinensis* and effectively avoids impact on breeding and aquaculture during sex identification. Moreover, the relatively short target sequences help prevent the detection failure due to environmental DNA degradation, enabling rapid and accurate identification of genetic sex in individual *Acipenser sinensis* of different ages. This method is applicable to *Acipenser sinensis* throughout their entire life cycle.

It should be noted that the terms "first", "second", and the like, as used in the specification of the present disclosure, are merely for distinguishing similar objects, and are not necessarily intended to describe a particular order or sequence. It should be understood that data used in such a way may be interchanged where appropriate, so that the embodiments of the present disclosure may be implemented in orders other than those illustrated or described herein.

Finally, it should be noted that the above embodiments are merely intended to illustrate the technical solution of the present disclosure, and are not intended to limit the same. Although the present disclosure has been described in detail with reference to the foregoing embodiments, it should be understood by those of ordinary skill in the art that the technical solutions described in the foregoing embodiments may be modified or equivalents may be substituted for some or all of the technical features thereof; and such modifications or substitutions do not make the essence of the corresponding technical solution deviate from the scope of the technical solution of each embodiment of the present disclosure.

## Claims

1. Use of a DNA fragment common to both male and female *Acipenser sinensis* and a female *Acipenser sinensis-specific* DNA fragment in genetic sex identification of *Acipenser sinensis,* wherein a nucleotide sequence of the DNA fragment common to both male and female *Acipenser sinensis* is as shown in SEQ ID NO:1, and a nucleotide sequence of the female *Acipenser sinensis-specific* DNA fragment is as shown in SEQ ID NO:2.

2. A primer set for amplifying a DNA fragment common to both male and female *Acipenser sinensis* and a female *Acipenser sinensis-specific* DNA fragment, comprising a first primer and a second primer for amplifying the DNA fragment common to both male and female *Acipenser sinensis,* and a third primer and a fourth primer for amplifying the female *Acipenser sinensis-specific* DNA fragment, wherein
a nucleotide sequence of the first primer is as shown in SEQ ID NO:3;
a nucleotide sequence of the second primer is as shown in SEQ ID NO:4;
a nucleotide sequence of the third primer is as shown in SEQ ID NO:5; and
a nucleotide sequence of the fourth primer is as shown in SEQ ID NO:6.

3. A kit for genetic sex identification of *Acipenser sinensis,* comprising the primer set of claim 2.

4. The kit according to claim 3, wherein the kit further comprises a negative control.

5. Use of the primer set of claim 2 or the kit of any one of claims 3-4 in genetic sex identification of *Acipenser sinensis.*

6. A method for genetic sex identification *of Acipenser sinensis,* comprising the following steps:
collecting a DNA sample from the *Acipenser sinensis* to be tested;
designing a first primer and a second primer for amplifying the DNA fragment common to both male and female *Acipenser sinensis,* and a third primer and a fourth primer for amplifying the female *Acipenser sinensis-specific* DNA fragment according to the DNA fragment common to both male and female *Acipenser sinensis* and a female *Acipenser sinensis-specific* DNA fragment of claim 1;
performing a first real-time quantitative polymerase chain reaction (PCR) detection on the DNA sample using the first primer and the second primer, and performing a second real-time quantitative PCR detection on the DNA sample using the third primer and the fourth primer; and
determining a genetic sex of the *Acipenser sinensis* to be tested based on results of the first and second real-time quantitative PCR detections.

7. The method according to claim 6, wherein the DNA sample is an environmental DNA sample.

8. The method according to claim 6 or 7, wherein a nucleotide sequence of the first primer is as shown in SEQ ID NO:3; and a nucleotide sequence of the second primer is as shown in SEQ ID NO:4.

9. The method according to claim 6 or 7, wherein a nucleotide sequence of the third primer is as shown in SEQ ID NO:5; and a nucleotide sequence of the fourth primer is as shown in SEQ ID NO:6.

10. The method according to any one of claims 6-9, wherein the determining a genetic sex of the *Acipenser sinensis* to be tested based on results of the first and second real-time quantitative PCR detections comprises:
when Ct values of both the first and second real-time quantitative PCR detection results are less than or equal to 36, it is determined that the genetic sex of the *Acipenser sinensis* is female; when a Ct value of the second real-time quantitative PCR detection result is greater than 36 or shows "undetermined", and a Ct value of the first real-time quantitative PCR detection result is less than or equal to 36, it is determined that the genetic sex of the *Acipenser sinensis* is male; and when Ct values of both the first and second real-time quantitative PCR detection results are greater than 36 or show "undetermined", it is determined that the DNA sample does not contain DNA from the *Acipenser sinensis.*
